# EUROPÄISCHE PATENTANMELDUNG

(11) **EP 2 050 740 A1**
(43) Veröffentlichungstag der Anmeldung: **22.04.2009**
(21) Anmeldenummer: 07118498.0
(22) Anmeldetag: 15.10.2007
(51) Int. Cl.: C07D 251/60

(54) **Verfahren zur Herstellung von Melamin**

(71) Anmelder: BASF SE, 67056 Ludwigshafen (DE)
(72) Erfinder: Die Erfindernennung liegt noch nicht vor

(57) **Zusammenfassung**

Verfahren zur Herstellung von Melamin durch Umsetzung von Harnstoff in Gegenwart eines Feststoff-Katalysators in einem oder in mehreren in Reihe geschalteten Reaktoren im Temperaturbereich von 370 °C bis 430 °C, Kühlen und Filtern des bei der Harnstoffumsetzung entstandenen Gases, Abtrennen des Melamins durch Desublimation und Rückführung eines Teiles des nach der Melaminabtrennung vorhandenen Gases ("Kreisgas") in den Reaktor oder die Reaktoren, dadurch gekennzeichnet, dass alle voranstehenden Schritte bei einem Druck im Bereich von 4 bar abs. bis 10 bar abs. durchgeführt werden.

## Beschreibung

Die Erfindung betrifft ein verbessertes Verfahren zur Herstellung von Melamin (2,4,6-Triamino-1,3,5-triazin) durch thermische Umsetzung von Harnstoff in Gegenwart eines Katalysators.

Melamin findet Verwendung zur Herstellung von Melamin-Harzen durch Umsetzung mit carbonylhaltigen Verbindungen. Die Harze werden unter anderem als Kunststoffe sowie in Farben und Lacken eingesetzt.

Die Herstellung von Melamin durch Zersetzung von Harnstoff ist eine bekannte Reaktion, die von der chemischen Industrie in mehreren Varianten benutzt wird. Einen Überblick findet man in Ullmann's Encyclopedia of Industrial Chemistry, 5th Edition 1990, Vol. A 16, Seiten 171 bis 185.

Prinzipiell wird zwischen dem Hochdruck- und dem Niederdruckverfahren unterschieden. Im folgenden sind die Druckangaben in bar (abs.).

Das Hochdruckverfahren wird üblicherweise bei Drücken von mehr als ca. 80 bar (abs.) und Temperaturen von über 370 °C durchgeführt, wobei die Melaminsynthese auf nicht katalytische Weise in einer Schmelze erfolgt.

Das Niederdruckverfahren wird in der Regel bei Drücken von ca. 1 bar (abs.) bis ca. 10 bar (abs.) und Temperaturen von 370 bis 430 °C in einer heterogenen Katalyse durchgeführt.

Nach bisherigem Kenntnisstand läuft die Reaktion im katalytischen Niederdruckverfahren dabei in zwei Schritten ab. Im ersten, endothermen Schritt zerfällt Harnstoff zu Ammoniak und Isocyansäure, die im zweiten, exothermen Schritt zu Melamin unter Freisetzung von Kohlendioxid trimerisiert. Die Gesamtreaktion (erster plus zweiter Schritt) ist endotherm.

Es existieren im Wesentlichen drei Varianten des Niederdruckverfahrens, auf die nachfolgend kurz eingegangen wird. Näheres entnehme man Ullmann's Encyclopedia of Industrial Chemistry, 5th Edition 1990, Vol. A 16, Seiten 171 bis 185.

Bei dem Verfahren der Linz-Chemie wird die Umsetzung in zwei Stufen durchgeführt. In der ersten Stufe wird Harnstoffschmelze bei 350 °C und 3,5 bar (abs.) in einer Sand-Wirbelschicht zu Ammoniak und Isocyansäure zersetzt. Anschließend wird in einem Festbettreaktor Isocyansäure bei 450 °C und Atmosphärendruck katalytisch zu Melamin umgesetzt. Der Katalysator ist dabei generell ein Aluminiumoxid-Katalysator. Das gasförmiges Melamin enthaltende Reaktionsgas wird mit Wasser abgekühlt ("gequencht") und so das Melamin aus dem Reaktorgas entfernt. Die wässrige Melamin-Suspension wird weiter relativ aufwändig in Fest-/Flüssigtrennstufen aufgearbeitet.

Das DSM-Stamicarbon-Verfahren ist ein einstufiges Verfahren, das bei ca. 7 bar (abs.) durchgeführt wird. Als Katalysatoren dienen Aluminiumsilicate oder zeolithhaltige Katalysatoren, die in einer Wirbelschicht eingesetzt werden. Als Wirbelgas dient reines Ammoniak, das aus der Aufarbeitungsstufe rückgewonnen wird. Das gasförmige Melamin enthaltende Reaktionsgas wird mit Wasser abgekühlt und das Melamin so aus dem Reaktorgas entfernt. Die wässrige Melamin-Suspension wird weiter relativ aufwändig in Fest-/Flüssigtrennstufen aufgearbeitet.

Bei dem bisherigen BASF-Verfahren schließlich wird bei niedrigem Druck (ca. 2 bar (abs.)) ebenfalls in der Wirbelschicht gearbeitet, wobei Aluminiumoxid- oder Aluminiumoxid-Siliciumdioxid-Katalysatoren, eingesetzt werden und Ammoniak und Kohlendioxid als Wirbelgas (auch Prozeßgas genannt) fungiert. Das heiße, gasförmige Melamin wird im Gegegensatz zum Linz-Chemie und DSM-Stamicarbon-Verfahren in einem sogenannten Kristallisierapparat (auch "Kristaller" genannt) durch Abkühlung auf ca. 200 °C desublimiert und fällt als ein feinkristallines Pulver an, welches dann im Gasstrom in einen Zyklon transportiert wird und dort vom Gas getrennt wird. Das Gas, welches neben Ammoniak und Kohlendioxid weitere Verunreinigungen enthalten kann, wird dann einem sogenannten Harnstoffwäscher zugeführt in dem es dann bei ca. 135 °C von den Verunreinigungen befreit und gekühlt wird. Ein Teil des Gases wird dann dem Reaktor als Gas für die Wirbelschicht ("Wirbelgas") zugeführt und tritt somit wieder in den Kreislauf ein. Ein anderer Teil des Gases wird in den Kristaller zum Kühlen eingespeist und ein letzter Teil des Gases verläßt den Kreislauf als Abgas.

Obwohl die bestehenden Verfahren großtechnisch eingesetzt werden, besteht doch noch Raum für Verbesserungen.

Die Hochdruckverfahren der Melaminsynthese haben verhältnismäßig geringe Einstrangkapazitäten von ca. 30.000 t/Jahr.

Die Einstrangkapazität ist die maximale Menge Melamin, die man pro Jahr aus einer Reaktions- und Aufarbeitungseinheit erhalten kann.

Auch das Niederdruckverfahren der BASF zum Beispiel hat noch eine relativ geringe Einstrangkapazität von ca. 40.000 t/Jahr.

Das Verfahren der DSM bei etwas höheren Drücken hat wohl eine größere Einstrangkapazität von ca. 80.000 t/Jahr. Nachteil dieses Verfahrens ist aber die, besonders apparativ und energetisch, aufwändige Abtrennung des Melamins durch Quenchen mit Wasser (nasse Aufarbeitung).

Nach wie vor sind noch nicht alle Vorgänge bei der Melaminsynthese nach den Verfahren des Standes der Technik verstanden. Viel Raum für Spekulationen bleibt zum Beispiel will man Ort, Natur und Reaktionsbedingungen für die Entstehung von Nebenprodukten und Folgeprodukten des Melamins erklären. Folgeprodukte des Melamins können, je nach Reaktionsbedingungen, beispielsweise die in Ullmann's Encyclopedia of Industrial Chemistry, 5th Edition 1990, Vol. A 16, Seiten 171 bis 185 genannten Melem, Melone und Melam sein, die vermutlich durch Reaktion von Melamin-Molekülen entstehen.

Weitere Nebenprodukte oder Folgeprodukte sind die ebenfalls in Ullmann's Encyclopedia of Industrial Chemistry, 5th Edition 1990, Vol. A 16, Seiten 171 bis 185 genannten Ammelin, Ammelid oder Cyanursäure (formaler Ersatz von einer, zwei oder drei -NH₂-Gruppen des Melamins durch -OH). Es ist nicht klar, ob sich diese Produkte aus kleineren Molekülen aufbauen, unter den Bedingungen, die im Melaminverfahren herrschen, und/oder durch (partielle) Hydrolyse fertig gebildeten Melamins entstehen.

Eine große Herausforderung ist es also, die geringe Menge Neben- und Folgeprodukte des Melamins von dem gewünschten Hauptprodukt Melamin abzutrennen oder die Neben- und Folgeprodukte erst gar nicht in nennenswerter Menge entstehen zu lassen, und dies alles bei hoher Jahreskapazität der großtechnischen Melaminsynthese.

Es besteht die Gefahr, dass sich Ausbeute und weitere Charakteristik (zum Beispiel Morphologie, Reinheit) des Melamins ändern, wenn man einen oder mehrere wesentliche Parameter (zum Beispiel Druck und Temperatur), im Melaminverfahren bei Synthese und/oder bei der Aufarbeitung ändert.

Die Aufgabe der Erfindung besteht darin, ein wirtschaftlich attraktiveres Verfahren zur Herstellung von Melamin aus Harnstoff bereitzustellen, ohne, dass sich die Produkteigenschaften, zum Beispiel Morphologie und Reinheit, des Melamins verschlechtern.

Die Aufgabe wurde gelöst mit dem Verfahren wie in den Ansprüchen definiert.

Das erfindungsgemäße Verfahren wird im folgenden näher beschrieben. In Grundzügen ist das erfindungsgemäße Verfahren analog dem in Ullmann's Encyclopedia of Industrial Chemistry, 5th Edition 1990, Vol. A 16, Seiten 171 bis 185 beschriebenen Verfahren der BASF.

Das Prozessgas der Melaminsynthese, bestehend aus Ammoniak und CO₂ im Massenverhältnis von ca. 1:1, entsteht bei der Melaminsynthese aus Harnstoff. Das Gas wird in der Regel um ca. 0,5 bis 2 bar verdichtet und anschließend auf 370 °C bis 430 °C erhitzt, bevor es in den Wirbelschichtreaktor als Wirbelgas eingespeist wird. Dieser Gasstrom kommt aus dem Harnstoffwäscher und, in einer bevorzugten Ausführungsform der Erfindung, direkt aus dem Gaskreislauf nach der Melaminabscheidung, wie unten näher beschrieben.

Die Reaktion Harnstoff zu Melamin kann in einem Reaktor oder in mehreren in Reihe geschalteten Reaktoren durchgeführt werden. Vorzugsweise wird die Reaktion in einem Reaktor oder in zwei in Reihe geschalteten Reaktoren durchgeführt. Wird die Reaktion in zwei in Reihe geschalteten Reaktoren durchgeführt, so kann sowohl im ersten als auch zweiten Reaktor der Katalysator in Form eines Wirbelbetts vorliegen, vorzugsweise liegt aber im zweiten Reaktor der Katalysator in Form eines Festbetts vor. Bei zwei in Reihe geschalteten Reaktoren spricht man auch von Hauptreaktor und Nebenreaktor.

Bevorzugt wird die Melaminherstellung in einem Reaktor durchgeführt.

Besonders bevorzugt wird die Melaminherstellung in einem Reaktor in einer Wirbelschicht durchgeführt.

Das heiße Wirbelgas bestehend aus Ammoniak und Kohlendioxid im Massenverhältnis von ca. 1:1 wird dem mit Katalysator gefüllten Reaktor zugeführt und fluidisiert den Feststoff.

Der im Wirbelschichtreaktor eingesetzte Katalysator ist ein üblicher Katalysator für die katalytische Melaminsynthese auf Basis von anorganischen Lewis-Säuren, vorzugsweise Lewis-saure Metalloxide, wie Aluminiumoxide oder Silizium-Aluminiumoxide.

Eine Harnstoffschmelze wird zusammen mit Ammoniak als Zerstäubungsgas in den Reaktor versprüht. Die Reaktortemperatur liegt im Bereich von 370 °C bis 430 °C, vorzugsweise im Bereich von 390 °C bis 420 °C, der Reaktordruck liegt im Bereich von 4 bar (abs.) bis 10 bar (abs.) vorzugsweise im Bereich von 5 bar (abs.) bis 8 bar (abs.).

Das Reaktionsgas verlässt den Reaktor über einen eingebauten Zyklonabscheider, der mitgerissene Feinanteile an Katalysator abtrennt und in die Wirbelschicht zurückführt. Am Reaktorausgang besteht das Prozessgas aus Melamin, Nebenprodukten, nicht umgesetzter Isocyansäure sowie Ammoniak und Kohlendioxid.

Der Umsatz von Harnstoff zu Melamin liegt im Bereich von 70 Gew.-% bis 97 Gew.-%, bezogen auf Harnstoff; vorzugsweise liegt der Umsatz von Harnstoff zu Melamin im Bereich von 80 Gew.-% bis 97 Gew.-%, bezogen auf Harnstoff, insbesondere liegt der Umsatz von Harnstoff zu Melamin im Bereich von 90 Gew.-% bis 97 Gew.-%, bezogen auf Harnstoff.

Das 370 °C bis 430 °C heiße Reaktionsgas aus dem Melaminreaktor oder gegebenenfalls Nachreaktor strömt durch einen Gaskühler, der das melaminhaltige Reaktionsgas auf eine Temperatur im Bereich von 320 °C bis 380 °C, vorzugsweise im Bereich von 330 °C bis 370 °C abkühlt. Bei diesen Temperaturen beginnen hochsiedende Nebenprodukte zu desublimieren und/oder sich auf dem von den Zyklonen im Wirbelschichtreaktor nicht zurückgehaltenen Katalysatorstaub niederzuschlagen. Das weiterhin gasförmige Melamin wird somit weitgehend von den höhersiedenden Nebenkomponenten gereinigt. Die Temperatur im Gaskühler bestimmt nach derzeitigem Kenntnisstand den Melemgehalt im Melamin und den Melamingehalt im nebenprodukthaltigen Katalysatorstaub. Die Gesamtmenge an weiterhin gasförmigen Nebenkomponenten (bezogen auf Melamin) im Reaktionsgas ist hierbei überraschenderweise nicht höher als in dem bisher üblichen Verfahren.

Das Reaktionsgas wird dann in Heißgasfilter eingespeist. Dort wird der von den Zyklonen im Wirbelschichtreaktor nicht zurückgehaltene Katalysatorstaub sowie die im Gaskühler desublimierten Nebenprodukte abgeschieden. Das aus dem Heißgasfilter austretende Gas hat üblicherweise eine Temperatur im Bereich von 320 °C bis 380 ° C und enthält in der Regel neben Melamin nur noch Spuren der hochsiedenden Nebenprodukte wie Melam, Melem und weiterhin nicht umgesetzte Isocyansäure. Der Katalysatorstaub wird aus dem Filter, üblicherweise über eine Druckschleuse, ausgeschleust und entsorgt.

Gaskühler und Heißgasfilter können redundant vorliegen und in A-B-Schaltung betrieben werden.

Das aus dem Heißgasfilter kommende Reaktionsgas wird im Kristaller bei einem Druck im Bereich von 4 bar (abs.) bis 7 bar (abs.), vorzugsweise im Bereich von 5 bar (abs.) bis 6 bar (abs.) mit dem im Harnstoffwäscher auf eine Temperatur im Bereich von 130 °C bis 150 °C, vorzugsweise 135 °C bis 150 °C abgekühltem und gewaschenem Gas (sogenanntes "Kühlgas", Zusammensetzung wie das Wirbelgas) gemischt und dadurch auf eine Temperatur im Bereich von 150 °C bis 250 °C, bevorzugt im Bereich von 200 °C bis 250 °C, besonders bevorzugt im Bereich von 210 °C bis 230 °C abgekühlt. Dabei desublimiert Melamin üblicherweise fast vollständig und fällt in der Regel als feines, weißes Pulver an.

So ist zum Beispiel die Kristallmorphologie des Melamins, gemessen mit der Methode der Rasterelektronenmikroskopie (REM), wie jene von derzeitigen kommerziellen Produkten. Die REM-Messung ist an sich bekannt. Zur Kristallmorphologiebestimmung des Melamins kann wie folgt vorgegangen werden. Das Melaminpulver wird auf ein leitfähiges Kohleklebepad gestreut und zur Erhöhung der Leitfähigkeit mit 2,5 nm Platin besputtert und dann die Oberfläche im REM abgebildet. Die Beschleunigungsspannung ist 3 kV, die Aufnahmen erfolgen mit dem Sekundärelektronendetektor unter einem Neigungswinkel von 13 Grad.

Auch die Partikelgrößenverteilung entspricht dem üblichen Standard. Die Partikelgrößenverteilung des Melamins kann durch Laserbeugung bestimmt werden (Methode nach ISO 13320). Hierzu wird das Melaminpulver mit einem Dispergierdruck von 2 bar trockendispergiert und beispielsweise in einem Analysegerät Mastersizer S (Firma Malvern) mit folgenden Meßparametern untersucht: Gasgeschwindigkeit 157 m/s; Streumodell 3$$A (Fraunhofer); Brennweite 300 mm; Strahllänge 10,00 mm.

So liegt z. B. der d-50-Wert (mittlerer Partikeldurchmesser) des mit dem erfindungsgemäßen Verfahren erhältlichen Melaminpulvers im Bereich von 10 µm bis 30 µm und der d-90-Wert im Bereich von 30 µm bis 50 µm.

Das desublimierte Melamin wird im allgemeinen pneumatisch in Zyklone gefördert, dort abgeschieden, ausgeschleust und zur Abfüllung weitergefördert.

Das aus dem Melaminzyklon austretende Reaktionsgas ("Kreisgas") hat eine Temperatur im Bereich von 200 °C bis 250 °C, vorzugsweise 210 °C bis 230 °C und enthält neben Ammoniak und Kohlendioxid noch Reste von Melaminstaub, Isocyansäure, und andere Nebenprodukte. Die Menge der Isocyansäure liegt im Bereich von 0,1 Vol.-‰ bis 2,0 Vol.-‰, vorzugsweise 0,1 Vol.-‰ bis 1,0 Vol.-‰.

In einer bevorzugten Ausführungsform wird ein Teil dieses heißen Kreisgases - vorzugsweise eine Gasmenge im Bereich von 5 % bis 50 %, besonders bevorzugt im Bereich von 10 % bis 20 %, jeweils bezogen auf die Gesamtgasmenge aus dem Zyklon - nach der Melaminabscheidung, ohne Durchgang durch den Harnstoffwäscher, wieder als Wirbelgas in den Melaminsynthesereaktor eingespeist ("warmes Wirbelgas").

Dieses warme Wirbelgas kann an jeder Stelle des Kreisgaswegs nach der Melaminabscheidung und vor der Kreisgaswäsche im Harnstoffwäscher abgezweigt werden. So kann das warme Wirbelgas in Strömungsrichtung direkt nach dem Kühlgasgebläse, welches üblicherweise das Kreisgas zum Harnstoffwäscher fördert, abgezweigt und in den Melaminsynthesereaktor gefördert werden. Bevorzugt wird das warme Wirbelgas allerdings in Strömungsrichtung vor dem Kühlgasgebläse, welches üblicherweise das Kreisgas zum Harnstoffwäscher fördert, abgezweigt und in den Melaminsynthesereaktor gefördert.

Der Kreisgasrest, also die Menge des Kreisgases nach der Melaminabscheidung die nicht erfindungsgemäß abgezweigt wurde, wird üblicherweise über ein Kühlgasgebläse zum Harnstoffwäscher gefördert. Dort wird der Kreisgasrest schließlich in einem mit Harnstoffumlauf betriebenen Wäscher auf eine Temperatur im Bereich von 130 °C bis 150 °C, vorzugsweise 135 °C bis 140 °C abgekühlt.

In diesem Apparat rekombiniert üblicherweise die verbliebene Isocyansäure mit Ammoniak zu Harnstoff, der wieder in den Melaminsynthesereaktor eingespeist wird.

Der aus dem Harnstoffwäscher austretende abgekühlte Kreisgasrest wird dann nach üblicher Reinigung, zum Beispiel Abtrennung von Harnstoff-Tröpfchen in Harnstoffabscheidungszyklonen, zum Teil als Kühlgas in den Kristallisierapparat rezykliert und zum Teil als Abgas des Gesamtprozesses entfernt.

Das erfindungsgemäße Verfahren zeichnet sich unter anderem durch folgende Vorteile aus:

Der Betrieb im erfindungsgemäßen höheren Druckbereich erlaubt eine deutliche Verringerung der spezifischen Apparatevolumina und ermöglicht damit erst den Bau einer Einstranganlage für Kapazitäten von üblicherweise mindestens 60.000 t/a. Durch die Anhebung des Druckniveaus sinken die Betriebsvolumenströme und damit der spezifische Energieverbrauch (Energieverbrauch pro Tonne Melamin) von Wirbelgas- und Kühlgasverdichter bei konstant gehaltenen Druckverlusten im System.

Durch eine bevorzugte Ausführungsform der Erfindung, nämlich durch den oben beschriebenen modifizierten Kreisgasweg, wird im Vergleich zum Stand der Technik die Kreisgasmenge, die bisher über das Kühlgasgebläse, den Harnstoffwäscher und die Harnstoffabscheidungszyklone geführt wird, um eine substantielle Menge reduziert.

Der Harnstoffumlauf über den Harnstoffwäscher wird in gleichem Maße reduziert, so dass neben den genannten Apparaten auch die Harnstoffpumpe(n) und Harnstoffwärmetauscher kleiner werden können.

Des weiteren verringert sich die Heizleistung des Wirbelgaserhitzers, da die Gaseintrittstemperatur im Bereich von 140 °C bis 200 °C auf im Bereich von 210 °C bis 250 °C erhöht wird.

Die Reduzierung der Investitionskosten und die Einsparungen im Stromverbrauch (Kühlgasgebläse und Harnstoffpumpe(n)) sowie im Erdgasverbrauch der Salzheizung stellen die Vorteile des modifizierten Gaswegs dar.

Das nach dem erfindungsgemäßen Verfahren erhältliche Melamin zeichnet sich durch hohe Reinheit aus und läßt sich unmittelbar weiterverarbeiten.

Die mit dem erfindungsgemäßen Verfahren erzielte Reinheit des Melamins, bestimmt mit der Methode der Trübungsmessung angelehnt an (DIN) EN ISO 7027, ist kleiner als 15 NTU (Nephelometric Turbitidy Unit). Die Trübungsmessung einer Melaminprobe kann zum Beispiel in einem HACH-Turbidimeter wie folgt durchgeführt werden. 14 g Melamin; 20,4 ml Formalin 30 %ig und 2 ml destilliertes Wasser werden vorgelegt. Die Probe wird elektrisch (Heizplatte oder Heizhaube) aufgeheizt und genau 30 Sekunden lang kochen lassen. Dann wird die Probe in einem auf 35 °C eingestellten Thermostat in genau 3 Minuten unter gelegentlichem Rühren auf ca. 50 °C abgekühlt, in eine Rundküvette gefüllt und im Turbidimeter gemessen.

## Patentansprüche

1. Verfahren zur Herstellung von Melamin durch Umsetzung von Harnstoff in Gegenwart eines Feststoff-Katalysators in einem oder in mehreren in Reihe geschalteten Reaktoren im Temperaturbereich von 370 °C bis 430 °C, Kühlen und Filtern des bei der Harnstoffumsetzung entstandenen Gases, Abtrennen des Melamins durch Desublimation und Rückführung eines Teiles des nach der Melaminabtrennung vorhandenen Gases ("Kreisgas") in den Reaktor oder die Reaktoren, **dadurch gekennzeichnet, dass** alle voranstehenden Schritte bei einem Druck im Bereich von 4 bar abs. bis 10 bar abs. durchgeführt werden.

2. Verfahren nach Anspruch 1, **dadurch gekennzeichnet, dass** die katalytische Harnstoffzersetzung und Melaminsynthese nur in einem Reaktor in der Wirbelschicht stattfindet.

3. Verfahren nach Anspruch 1 oder 2, **dadurch gekennzeichnet, dass** man nach der Melaminabtrennung ein Teil des Rückgases abzweigt und ohne Durchgang durch den Harnstoffwäscher in den Reaktor als "Wirbelgas" einspeist.

4. Verfahren nach Anspruch 3, **dadurch gekennzeichnet, dass** man das Rückgas in Strömungsrichtung vor dem Kühlgasgebläse abzweigt.

5. Verfahren nach den Ansprüchen 1 bis 4 **dadurch gekennzeichnet, dass** man die Desublimation bei einer Temperatur im Bereich von 150 °C bis 250 °C durchführt.
